(19) Europäisches Patentamt
European Patent Office
Office européen des brevets

(11) Veröffentlichungsnummer: **0 370 415 B1**

# (12) EUROPÄISCHE PATENTSCHRIFT

(45) Veröffentlichungstag der Patentschrift: **02.06.93**

(51) Int. Cl.⁵: **C07D 453/02**, A61K 31/435

(21) Anmeldenummer: **89121387.8**

(22) Anmeldetag: **18.11.89**

## (54) Ouinuclidine, ihre Verwendung als Arzneimittel und Verfahren zu ihrer Herstellung.

(30) Priorität: **22.11.88 DE 3839385**

(43) Veröffentlichungstag der Anmeldung:
**30.05.90 Patentblatt 90/22**

(45) Bekanntmachung des Hinweises auf die Patenterteilung:
**02.06.93 Patentblatt 93/22**

(84) Benannte Vertragsstaaten:
**AT BE CH DE ES FR GB GR IT LI LU NL SE**

(56) Entgegenhaltungen:
**DE-A- 1 938 546**

**HELVETICA CHIMICA ACTA, Band 57, 1974 W.ECKHARDT et al. "Synthese und BasizitUt 4-hetero- substituierter Chinuclidine" Seiten 2339-2345**

**HETEROCYCLES, Band 25, 1987 P.L.STOTTER et al. "Ouinu- clidine-boranes as inter- mediates information and isolation of functio- nalized quinuclidine systems" Seiten 251-258**

(73) Patentinhaber: **BOEHRINGER INGELHEIM KG**

**W-6507 Ingelheim am Rhein(DE)**

(84) Benannte Vertragsstaaten:
**BE CH DE ES FR GR IT LI LU NL SE AT**

(73) Patentinhaber: **BOEHRINGER INGELHEIM INTERNATIONAL G.M.B.H.**

**W-6507 Ingelheim am Rhein(DE)**

(84) Benannte Vertragsstaaten:
**GB**

(72) Erfinder: **Walther, Gerhard, Dr.**
**Pfarrer-Heberer-Strasse 37**
**W-6530 Bingen(DE)**
Erfinder: **Weber, Karl-Heinz, Dr.**
**Kaiser-Karl-Strasse 11**
**W-6535 Gau-Algesheim(DE)**
Erfinder: **Stransky, Werner, Dr.**
**Im Hippel 24**
**W-6535 Gau-Algesheim(DE)**
Erfinder: **Kuhn, Franz, Dr.**
**Beethovenstrasse 11**
**W-6535 Gau-Algesheim(DE)**
Erfinder: **Müller, Enzio, Prof. Dr.**
**Grundstrasse 22**
**W-6507 Ingelheim am Rhein(DE)**
Erfinder: **Ensinger, Helmut, Dr.**
**Magdeburger Strasse 54**
**W-6507 Ingelheim am Rhein(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 370 415 B1

Rank Xerox (UK) Business Services
(3.10/3.6/3.3.1)

## Beschreibung

Die Erfindung betrifft Quinuclidine, Verfahren zu ihrer Herstellung und ihre Verwendung als Arzneimittel. Die Quinuclidine entsprechen der allgemeinen Formel

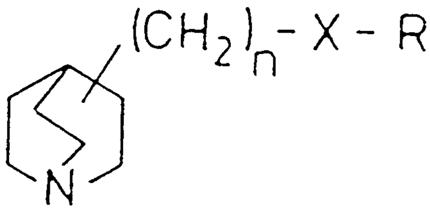

I

worin

- R Alkyl, Alkenyl oder Alkinyl;
- X Sauerstoff oder Schwefel und
- n 0, 1 oder 2 bedeuten sowie

gegebenenfalls in Form ihrer Racemate, ihrer Enantiomere, ihrer Diastereomere und ihrer Gemische sowie gegebenenfalls ihre pharmakologisch unbedenkliche Säureadditionssalze wie auch ihre Quartärsalze.

Als Alkylgruppen im Sinne dieser Erfindung werden verzweigte oder unverzweigte Alkylreste mit 1 bis 6 Kohlenstoffatomen, wie z. B. Methyl, Ethyl, Propyl, Butyl, Pentyl und Hexyl wie auch deren verzweigte Isomere, wie z.B. iso-Propyl, iso-Butyl oder, tert.-Butyl, verstanden; unter Alkenylgruppen werden verzweigte oder unverzweigte Alkenylreste mit 3 bis 6 Kohlenstoffatomen - wie z.B. Propenyl, Butenyl, Pentenyl und Hexenyl - mit einer Doppelverbindung; und unter Alkinylgruppen werden verzweigte oder unverzweigte Alkinylreste mit 3 bis 6 Kohlenstoffatomen - wie z.B. Propinyl, Butinyl und Pentinyl - mit einer Dreifachbindung verstanden. Bevorzugt sind solche Alkenyl- oder Alkinylreste, in denen die Doppel- bzw. Dreifachbindung endständig angeordnet ist.

Bevorzugte Verbindungen der allgemeinen Formel I sind solche, worin X Sauerstoff, $n = 0$ oder 1 und der Substituent $(CH_2)_nXR$ in der 3-Position eine Alkylgruppe mit 1 bis 3 Kohlenstoffatomen, eine Alkenylgruppe mit 3 oder 4 Kohlenstoffatomen oder eine Alkinylgruppe mit 3 oder 4 Kohlenstoffatomen bedeuten.

Einige Verbindungen der allgemeinen Formel sind bekannt. So beschreiben L. Stotter et al. in Heterocycles, Vol. 25 (1987) Seite 251 ff. die in 3-Position substituierten Methyl-, Ethyl- und Allylether des Quinuclidins. Aus Helvetica Chim Acta Vol 57 (1974) Seiten 2339 ff ist der 4-Methylthio- und 4-Methylether des Quinuclidins bekannt. Die Autoren offenbaren nicht die Verwendung dieser Substanzen als Arzneimittel. Ebenfalls bekannt ist der 3-Methylethylether wie auch der 3-Methylethylthioether.

Die Verbindungen der allgemeinen Formel I können in Analogie zu den von Stotter beschriebenen Verfahren synthetisiert werden. Ausgehend von den N-geschützten Hydroxy bzw. Mercapto - Quinuclidinen erhält man die erfindungsgemäßen Verbindungen durch Deprotonierung mit starken Basen und anschließender Umsetzung mit einem Alkylierungsreagenz der allgemeinen Formel Y-R, worin R wie zuvor definiert ist und Y eine leicht abspaltbare Austrittgruppe - wie z.B. Halogen oder, p-Toluolsulfonat - bedeutet. Die Reaktion wird in polaren inerten organischen Lösungsmitteln, wie z.B. Dimethylformamid, Tetrahydrofuran, Dioxan u.a., durchgeführt.

Während die Deprotonierung und Überführung der Hydroxyverbindung in ein Metallsalz, bevorzugt bei Raumtemperatur oder leicht erhöhter Temperatur durchgeführt wird, erfolgt die anschließende Alkylierung bevorzugt unter Eiskühlung.

Nach erfolgter Umsetzung wird die Schutzgruppe abgespalten und die Verbindungen gegebenenfalls in ihre Säureadditionssalze oder Quartär-Verbindungen überführt, die Reaktionsbedingungen hierzu sind bekannt, bevorzugte Quartärverbindungen sind die Methojodide und Methobromide..

Bevorzugte Reagenzien zur Deprotonierung sind Natriumhydrid, Natriumamid, Alkalialkoholate, wie z.B. Kalium-tert.-Butylat.

Die erfindungsgemäßen Verbindungen besitzen -in Abhängigkeit der Position der Seitenkette- ein oder zwei chirale Zentren. Die Trennung der Racemate kann nach bekannten Methoden, wie z.B. durch chromatographische Trennverfahren oder durch Kristallisation, gegebenenfalls unter Verwendung chiraler oder prochiraler Hilfsstoffe erfolgen. Alternativ kann die Synthese ausgehend von optisch aktiven Ausgangsverbindungen erfolgen.

Wolfgang Eckert et al, Helvetica Chimica Acta, Vol 57 (1974), 2339 beschreiben ein Verfahren zur Herstellung von Thioethern der Quinuclidine, das sich in erster Linie zur Synthese von Derivaten in der 4-Position eignet.
Die Herstellung der als Ausgangsverbindungen geeigneten Hydroxy - oder Mercaptoquinuclidine der allgemeinen Formel I - worin X Sauerstoff oder Schwefel und R Wasserstoff bedeuten erfolgen nach an sich bekannten Analogieverfahren, wie sie beispielsweise bei K. B. Shaw, Canadian Journal of Chemistry Vol. 43, 3112, (1965) und in der DE-OS 19 38 546 beschrieben sind. Die Umsetzung zu den entsprechenden Thioethern gelingt - ausgehend von den Mercaptanen - oft schon in schwach alkalischer Lösung, vgl. A Schöberl und A. Wagner in Houben-Weyl Methoden der organischen Chemie, Georg Thieme Verlag Stuttgart (1955), 4. Auflage, Band 9, Seite 93 ff, wobei auf die bei den Sauerstoffethern erforderliche Schutzgruppe verzichtet werden kann).

Die Verbindungen der allgemeinen Formel I besitzen wertvolle pharmakologische Eigenschaften. So zeigen die Verbindungen in Bindungsstudien Affinitäten zu muskarinen Rezeptoren und muskarin-agonistische GTP-Shifts (GTP = Guanosintriphosphat). (Birdsall, N.I.M., E.C. Hulme and I.M. Stockton 1984 in T.I.P.S. Supplement, Proc. Internat. Symposium on Subtypes of Muscarinic Receptors, Ed. Hirschowitz, Hammer, Giacchetti, Klirns, Levine; Elsevier p. 4 - 8).

Die Rezeptorbindungsstudien wurden gemäß dem nachstehenden Literaturzitat ausgeführt [A. Closse, H. Bittiger, D. Langenegger und A. Wahner; Naunyn-Schmiedeberg's Arch. Pharmacol. 335, 372 - 377 (1987)].

Tabelle A: Rezeptorbindungsstudien

Radioligand: L(+)cis-[2-Methyl-$^3$H]-N,N,N-trimethyl-1, 3-dioxolan-4-methanammonium-jodid NET-647, Fa. NEN (New England Nuclear DU PONT).
Organ: Cerebraler Cortex (Ratte)

Tabelle A

| Beispiel | R | Ki [nMol/l] |
|---|---|---|
| 1 | $-CH_2-C\equiv CH$ | 159 |
| 3 | $-CH_3$ | 8300 |
| 4 | $-C_2H_5$ | 410 |
| 2 | $-nC_3H_7$ | 220 |

In pharmakologischen Testmodellen wurde in vitro und in vivo eine cholinomimetische Wirkung nachgewiesen. So zeigt beispielsweise 3-(2-Propinyloxy)-1-azabicyclo-[2,2,2]octan-fumarat in einer Dosis von 3 mg/kg i.v. im EEG (Elektroenzephalogramm) des wachen Kaninchens eine für Cholinomimetika typische Arousal-Reaktion.

Als muskarine Agonisten (Cholinomimetika) sind die Substanzen zur Therapie von Krankheiten bei einer Unterfunktion des cholinergen Systems geeignet.

Aufgrund pharmakologischer Befunde sind die Verbindungen z.B. zur Behandlung nachfolgend genannter Krankheiten geeignet: Morbus Alzheimer, senile Demenz, kognitive Störungen und außerdem können die Verbindungen zur Verbesserung der Gedächtnisleistung eingesetzt werden.

Quartäre Verbindungen der allgemeinen Formel I eignen sich besonders zur peripheren Anwendung, so z.B. zur Glaukombehandlung.

Die Verbindungen der allgemeinen Formel I können allein oder in Kombination mit anderen erfindungsgemäßen Wirkstoffen, gegebenenfalls auch in Kombination mit weiteren pharmakologisch aktiven Wirkstoffen, z.B. einen peripheren cholinergen Blocker, Cerebroaktivatoren zur Anwendung gelangen. Geeignete Anwendungsformen sind beispielsweise Tabletten, Kapseln, Zäpfchen, Lösungen, Säfte, Emulsionen oder dispersible Pulver.
Entsprechende Tabletten können beispielsweise durch Mischen des oder der Wirkstoffe mit bekannten Hilfsstoffen, beispielsweise inerten Verdünnungsmitteln, wie Calciumcarbonat, Calciumphosphat oder Milchzucker, Sprengmitteln, wie Maisstärke oder Alginsäure, Bindemitteln, wie Stärke oder Gelatine, Schmiermitteln, wie Magnesiumstearat oder Talk, und/oder Mitteln zur Erzielung des Depoteffektes, wie Carboxymethylcellulose, Celluloseacetatphthalat, oder Polyvinylacetat erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Kollidon oder Schellack, Gummi arabicum, Talk Titandioxid oder Zucker, hergestellt werden. Zur Erzielung eines Depoteffektes oder zur Vermeidung von Inkompatibilitäten kann der Kern auch aus mehreren Schichten bestehen. Desgleichen kann auch die Drageehülle zur Erzielung eines Depoteffektes aus mehreren Schichten bestehen, wobei die oben bei den Tabletten erwähnten Hilfsstoffe verwendet werden können.

Säfte der erfindungsgemäßen Wirkstoffe beziehungsweise Wirkstoffkombinationen können zusätzlich noch ein Süßungsmittel, wie Saccharin, Cyclamat, Glycerin oder Zucker sowei ein geschmacksverbesserndes Mittel, z.B. Aromastoffe, wie Vanillin oder Orangenextrakt, enthalten. Sie können außerdem Suspendierhilfsstoffe oder Dickungsmittel, wie Natriumcarboxymethylcellulose, Netzmittel, beispielsweise Kondensationsprodukte von Fettalkoholen mit Ethylenoxid, oder Schutzstoffe, wie p-Hydroxybenzoate, enthalten.

Injektionslösungen werden in üblicher Weise, z.B. unter Zusatz von Konservierungsmitteln, wie p-Hydroxybenzoaten, oder Stabilisatoren, wie Alkalisalzen der Ethylendiamintetraessigsäure hergestellt und in Injektionsflaschen oder Ampullen abgefüllt.

Die eine oder mehrere Wirkstoffe beziehungsweise Wirkstoffkombinationen enthaltenden Kapseln können beispielsweise hergestellt werden, indem man die Wirkstoffe mit inerten Trägern, wie Milchzucker oder Sorbit, mischt und in Gelatinekapseln einkapselt.

Geignete Zäpfchen lassen sich beispielsweise durch Vermischen mit dafür vorgesehenen Trägermitteln, wie Neutralfetten oder Polyäthylenglykol beziehungsweise dessen Derivaten, herstellen.

Die therapeutisch wirksame Einzeldosis liegt im Bereich zwischen 1 und 100 mg.

Die nachfolgenden Beispiele illustrieren die vorliegende Erfindung.

Pharmazeutische Formulierungsbeispiele

| A) Tabletten | pro Tablette |
|---|---|
| Wirkstoff | 80 mg |
| Milchzucker | 140 mg |
| Maisstärke | 240 mg |
| Polyvinylpyrrolidon | 15 mg |
| Magnesiumstearat | 5 mg |
| | 480 mg |

Der feingemahlene Wirkstoff, Milchzucker und ein Teil der Maisstärke werden miteinander vermischt. Die Mischung wird gesiebt, worauf man sie mit einer Lösung von Polyvinylpyrrolidon in Wasser befeuchtet , knetet, feuchtgranuliert und trocknet. Das Granulat, der Rest der Maisstärke und das Magnesiumstearat werden gesiebt und miteinander vermischt. Das Gemisch wird zu Tabletten geeigneter Form und Größe verpreßt.

| B) Tabletten | pro Tablette |
|---|---|
| Wirkstoff | 60 mg |
| Maisstärke | 190 mg |
| Milchzucker | 55 mg |
| Mikrokristalline Cellulose | 35 mg |
| Polyvinylpyrrolidon | 15 mg |
| Natrium-carboxymethylstärke | 23 mg |
| Magnesiumstearat | 2 mg |
| | 380 mg |

Der feingemahlene Wirkstoff, ein Teil der Maisstärke, Milchzucker, mikrokristalline Cellulose und Polyvinylpyrrolidon werden miteinander vermischt, die Mischung gesiebt und mit dem Rest der Maisstärke und Wasser zu einem Granulat verarbeitet, welches getrocknet und gesiebt wird. Dazu gibt man die Natrium carboxy methylstärke und das Magnesiumstearat, vermischt und verpreßt das Gemisch zu Tabletten geeigneter Größe.

| C) Ampullen | |
|---|---|
| Wirkstoff | 20 mg |
| Natriumchlorid | 10 mg |
| bidestilliertes Wasser q.s. ad | 1,0 ml |

Herstellung:

Der Wirkstoff und das Natriumchlorid werden in bidestilliertem Wasser gelöst und die Lösung in Ampullen steril abgefüllt.

| D) Tropfen | |
|---|---|
| Wirkstoff | 5,0 g |
| p-Hydroxybenzoesäuremethylester | 0,1 g |
| p-Hydroxybenzoesäurepropylester | 0,1 g |
| entmineralisiertes Wasser q.s. ad | 100,0 ml |

Herstellung:

Der Wirkstoff und die Konservierungsmittel werden in demineralisiertem Wasser gelöst und die Lösung filtriert und in Flaschen zu je 100 ml abgefüllt.

Beispiel 1

3-(2-Propinyloxy)-1-azabicyclo[2,2,2]octan,

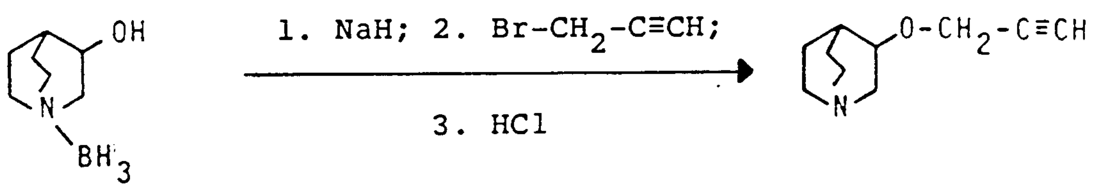


14,1 g (0,1 Mol) 3-Hydroxy-1-azabicyclo[2,2,2]octan Boran-Komplex werden in 140 ml Dimethylformamid bei Raumtemperatur unter Stickstoff mit 4 g Natriumhydrid-Dispersion (60 %ig in Öl) in das Natriumsalz überführt. Nach beendeter Wasserstoffentwicklung wird die Reaktionsmischung unter Eiskühlung mit einer Lösung von 14,28 g (0,12 Mol) Propargylbromid in 10 ml Toluol versetzt, drei Stunden bei Raumtemperatur gerührt und nach Zugabe von 5 ml Ethanol am Rotationsverdampfer im Vakuum eingeengt. Der Rückstand wird zwischen 10 %iger Kochsalzlösung und Ether verteilt. Die organische Phase wird über wasserfreiem Natriumsulfat getrocknet und eingedampft. Das zurückbleibende dunkelbraune Öl wird mit 75 ml Tetrahydrofuran und 15 ml Aceton aufgenommen. In die entstehende Lösung tropft man unter Eiskühlung 30 ml 4N Salzsäure (Gasentwicklung). Man läßt noch eine Stunde bei Raumtemperatur nachreagieren und destilliert die organischen Lösungsmittel ab. Der Rückstand wird mit wenig Wasser verdünnt und nacheinander mit Petroläther (40 - 60°) und Ether ausgeschüttelt. Die wäßrige Phase wird mit 40 %iger Kaliumcarbonat-Lösung alkalisch gestellt und mit Ether extrahiert. Die Etherlösung wird über wasserfreiem Natriumsulfat getrocknet und eingeengt. Man erhält 6,9 g (41,8 % d. Th.) 3-(2-Propinyloxy)-1-azabicyclo-[2,2,2]octan Base, die mit einem Äquivalent Fumarsäure in das Fumarat (Fp. 138 - 140° C; aus Ethanol/Ether) überführt wird.

| $C_{10}H_{15}NO \times C_4H_4O_4$ (281,31) | | | |
|---|---|---|---|
| ber. | C 59,77 | H 6,81 | N 4,98 |
| gef. | 59,86 | 6,76 | 4,95 |

$^1$H NMR ($CH_3OD$): σ 6,66 (s, 2H, FU C = C CH); 4,25 (d, 2H, J = 3 Hz, $OCH_2$); 4,11 (m, 1H, OCH); 3,67 - 3,12 (m, 6H, 3 $NCH_2$); 2,93 (t, 1H, J = 3 Hz, =CH); 2,46 - 1,70 (m, 5H, $-CH_2-CH-CH_2-$).

In analoger Weise werden hergestellt:

Beispiel 2

3-(n-Propyloxy)-1-azabicyclo[2,2,2]octan

Fp. 120 - 121° C ($CH_3CN$), Fumarat
Die Substanz enthält nach Mikroanalyse und NMR-Spektrum 1,5 Mol Fumarsäure.

| $C_{10}H_{19}NO \times 1{,}5\ C_4H_4O_4$ (343,38) | | | |
|---|---|---|---|
| ber. | C 55,97 | H 7,34 | N 4,08 |
| gef. | 55,67 | 7,51 | 4,09 |

Beispiel 3

3-Methoxy-1-azabicyclo[2,2,2]octan

Fp. 72 - 74° C (Essigester/Ether), Maleinat
Die Substanz enthält nach Mikroanalyse und NMR-Spektrum 1,5 Mol Maleinsäure.

| $C_8H_{15}NO \times 1{,}5\ C_4H_4O_4$ (315,33) | | | |
|---|---|---|---|
| ber. | C 53,66 | H 6,73 | N 4,83 |
| gef. | 53,33 | 6,71 | 4,44 |

Beispiel 4

3-Ethoxy-1-azabicyclo[2,2,2]octan

Fp. 96 - 98° C (Aceton/Ether), Maleinat

| $C_9H_{17}NO \times C_4H_4O_4$ (271,33) | | | |
|---|---|---|---|
| ber. | C 57,55 | H 7,80 | N 5,16 |
| gef. | 57,40 | 8,01 | 5,18 |

$^1$H NMR ($CD_3OD$): σ 6,23 (s, 2H, MA = CH); 3,55 (qu, 2H, J = 7 Hz, $OCH_2$): 3,98 - 3,08 (m, 7H, 3 $N-CH_2$, 1 CHO); 2,43 - 1,68 (m, 5H, $-CH_2-CH-CH_2$).

Beispiel 5

3-Allyloxy-1-azabicyclo[2,2,2]octan

Fp. 129 - 132° C (Methanol/Ether), Fumarat

| $C_{10}H_{17}NO$ x $C_4H_4O_4$ (283,33) | | | |
|---|---|---|---|
| ber.<br>gef. | C 59,35<br>59,19 | H 7,47<br>7,64 | N 4,94<br>5,03 |

$^1H$ NMR ($CD_3OD$): σ 6,66 (S, 2H, FU = CH); 5,90 (m, 1H, =CH); 5,21 (m, 2H, =$CH_2$); 4,03 (m, 2H, $OCH_2$); 4,14 - 3,09 (m, 7H, 3 N-$CH_2$); 2,45 - 1,69 (m, 5H, -$CH_2$-CH-$CH_2$-).

Beispiel 6

Racematspaltung von 3-(2-Propinyloxy)-1-azabicyclo-[2,2,2]octan

a.) (+)-3-(2-Propinyloxy)-1-azabicyclo[2,2,2]octanfumarat

16 g (96,82 mmol) 3-(2-Propinyloxy)-1-azabicyclo[2,2,2]octan Base und 18,22 g (48,41 mmol) (-)-Dibenzoyl-L-weinsäuremonohydrat werden in 160 ml Acetonitril unter Erwärmen gelöst. Man erhält durch mehrfache Kristallisation das entsprechende Dibenzoyltartrat vom Fp. 149 - 150° C (Zers.)
$[\alpha]_D$ - 43,1° (c = 1; $H_2O$)
Das so erhaltene Salz wird in wenig Wasser gelöst, mit wässeriger 40 % Kaliumcarbonatlösung alkalisch gestellt und 2 mal mit Essigester ausgeschüttelt. Die vereinigten organischen Phasen werden über wasserfreiem Natriumsulfat getrocknet und eingeengt. Vom erhaltenen farblosen Öl werden 0,8 g (4,9 mmol) und 0,56 g (4,9 mmol) Fumarsäure in wenig Ethanol gelöst. Durch Zugabe von Ether erhält man die Titelverbindung vom
Fp. 145 - 147°C.
$[\alpha]_D$ + 35,2° (c = 1; $H_2O$)

| | | | |
|---|---|---|---|
| ber.: | 59,77 C | 6,81 H | 4,98 N |
| gef.: | 59,75 C | 6,99 H | 5,15 N |

b) (-)-3-(2-Propinyloxy)-1-azabicyclo[2,2,2]octanfumarat

Die unter a) beschriebene Mutterlauge wird eingeengt und in die Base überführt.
8 g (0,048 Mol) der Base und 9,11 g (0,024 Mol) (+)-Dibenzoyl-D-weinsäuremonohydrat werden in Acetonitril gelöst. In Analogie zu a) erhält man das Benzoyltartrat (Fp: 145 - 150°C Z $[\alpha]_D$ + 43,4° (c = 1; $H_2O$)) und daraus 2,1 g der Titelverbindung vom Fp. 145 - 147°C.
$[\alpha]_D$ - 35° (c = 1; $H_2O$)

| | | | |
|---|---|---|---|
| ber.: | 59,77 C | 6,81 H | 4,98 N |
| gef.: | 59,79 C | 7,05 H | 4,97 N |

Beispiel 7

3-(2-Butinyloxy)-1-azabicyclo[2,2,2]octan

Fp. 131 - 132 ° C (Acetonitril), Fumarat

| $C_{11}H_{17}NO$ x $C_4H_4O_4$ (295,34) | | | |
|---|---|---|---|
| ber.<br>gef. | C 61,00<br>60,92 | H 7,17<br>7,40 | N 4,74<br>4,70 |

Beispiel 8

3-(2-Methyl-propyloxy)-1-azabicyclo[2,2,2]octan

Fp. 120 - 122 ° C (Methanol/Ether), Fumarat
Die Substanz enthält nach Mikroanalyse und NMR Spektrum 1,5 Mol Fumarsäure

| $C_{11}H_{21}NO$ x 1,5 $C_4H_4O_4$ (357,41) | | | |
|---|---|---|---|
| ber.<br>gef. | C 57,13<br>57,48 | H 7,61<br>7,66 | N 3,92<br>4,10 |

Beispiel 9

3-Methoxymethyl-1-azabicyclo[2,2,2]octan

Fp. 133 - 136 ° C (Ethanol/Ether), Fumarat

| $C_9H_{17}NO$ x $C_4H_4O_4$ (271,32) | | | |
|---|---|---|---|
| ber.<br>gef. | C 57,55<br>57,20 | H 7,80<br>7,87 | N 5,15<br>5,12 |

Beispiel 10

3-Ethoxymethyl-1-azabicyclo[2,2,2]octan

Fp. 73 - 77 ° C (Ethanol/Ether) Fumarat

| $C_{10}H_{19}NO$ x $C_4H_4O_4$ x $H_2O$ (303,36) | | | |
|---|---|---|---|
| ber.<br>gef. | C 55,43<br>55,15 | H 8,31<br>8,19 | N 4,62<br>4,52 |

Beispiel 11

3-(2-Propinyloxymethyl)-1-azabicyclo[2,2,2]octan

Fp. 88 -90 ° C (Acetonitril), Oxalat
Die Substanz kristallisiert mit 1,5 Mol Oxalsäure.

| $C_{11}H_{17}NO$ x 1,5 $C_2H_2O_4$ (314,32) | | | |
|---|---|---|---|
| ber.<br>gef. | C 53,50<br>53,67 | H 6,41<br>6,66 | N 4,46<br>4,59 |

Beispiel 12

3-Allylmercapto-1-azabicyclo[2,2,2]octan

2,86 g (0,02 Mol) 3-Mercapto-1-azabicyclo[2,2,2]octan werden in 30 ml Dimethylformamid gelöst und unter Stickstoff durch Zugabe von 2,24 g (0,02 Mol) Kaliumtert - butylat in das Kaliumsalz überführt. Anschließend werden unter Rühren und Eiskühlung 2,42 g (0,02 Mol) Allylbromid hinzugefügt. Die Reaktionsmischung wird 45 Minuten bei Raumtemperatur gerührt, mit verdünnter Salzsäure angesäuert und im Vakuum eingeengt. Der Rückstand wird in wenig Wasser gelöst, mit 40 %iger Kaliumcarbonat-Lösung alkalisch gestellt und mit Ether extrahiert. Die organische Phase wird getrocknet und eingedampft. Eine methanolische Lösung des Rückstandes wird über eine Kieselgelsäule filtriert. Nach dem Abdampfen des Lösungsmittels erhält man 2,9 g Rohbase, die in das Oxalat überführt werden.
Fp. 90 - 92° C (Acetonitril/Ether)

| $C_{10}H_{17}NS$ x 1,5 $C_2H_2O_4$ (318,38) | | | | |
|---|---|---|---|---|
| ber.<br>gef. | C 49,04<br>48,89 | H 6,33<br>6,42 | N 4,10<br>4,44 | S 10,07<br>10,21 |

Beispiel 13

3-Ethylmercapto-1-azabicyclo[2,2,2]octan

Fp. 101 - 102° C (Acetonitril/Ether), Oxalat

| $C_9H_{17}NS$ x 1,5 $C_2H_2O_4$ (306,37) | | | | |
|---|---|---|---|---|
| ber.<br>gef. | C 47,05<br>47,16 | H 6,58<br>6,92 | N 4,57<br>4,70 | S 10,47<br>10,63 |

Beispiel 14

(-)-3-(Propinyloxy)-1-azabicyclo[2,2,2]octan

Eine Suspension von 12,72 g (0,1 Mol) (R)-3-Quinuclidinol [Lit. B. Ringdahl et al., Acta Pharma Sueg. 16, 281 ff. (1979)] in 200 ml absolutem Tetrahydrofuran wird unter Stickstoff und Rühren bei 0°C mit 100 ml einer 1 molaren Lösung von Borantetrahydrofuran-Komplex in Tetrahydrofuran versetzt. Die Reaktionslösung wird noch eine Stunde bei Raumtemperatur nach gerührt und anschließend im Vakuum eingedampft. Der ölige Rückstand wird zwischen Methylenchlorid und gesättigter Kochsalzlösung verteilt. Die organische Phase wird über wasserfreiem Natriumsulfat getrocknet und eingeengt. Das zurückbleibende Harz wird mit Cyclohexan zur Kristallisation gebracht. Man erhält 12 g (85,1 % d. Th.) (R)-3-Quinuclidinol-Boran-Komplex; Fp. 186 - 189°C (Zers.)

Durch Umsetzung mit Propargylbromid analog Beispiel 1 erhält man (-)-3-(2-Propinyloxy)-1-azabicyclo-[2,2,2]octan.Die rohe Base wird durch Destillation [Kp. 121°/20 mbar] gereinigt und in das sauere Fumarat überführt. Hierzu löst man die Base mit einem Äquivalent Fumarsäure in Methanol und fällt das Salz durch Zugabe von Äther.
Fp. 149 - 151°C; $[\alpha]_D^{23}$ - 36,3° (c = 1; $H_2O$)

Beispiel 15

(+)-3-(Propinyloxy)-1-azabicyclo[2,2,2]octan

Ausgehend von (S)-3-Quinuclidinol erhält man analog Beispiel 14 die Titelverbindung als Hydrogenfumarat.
Fp. 149 - 151 °C; $[\alpha]_D^{23}$ + 36,5 ° (c = 1; $H_2O$).

Beispiel 16

(-)-3-Ethoxy-1-azabicyclo[2,2,2]octan

erhält man analog Beispiel 14 aus (R)-3-Quinuclidinol und Ethyljodid. Kp. 88 - 90 °C / 20 mb; Hydrogenfumarat:
Fp. 148 - 149 °C; $[\alpha]_D^{23}$ - 27,2 ° (c = 1; $H_2O$).

| $C_9H_{17}NO$ x $C_4H_4O_4$ (271,32) | | | |
|---|---|---|---|
| ber. | C 57,55 | H 7,80 | N 5,16 |
| gef. | 57,61 | 7,96 | 5,29 |

Das (+)-drehende Enantiomere wird entsprechend aus ((S)-3-Quinuclidinol erhalten.
Fp. 148 - 149 °C; $[\alpha]_D^{23}$ + 27,4 ° (c = 1; $H_2O$)

## Patentansprüche

**1.** Quinuclidine der allgemeinen Formel I

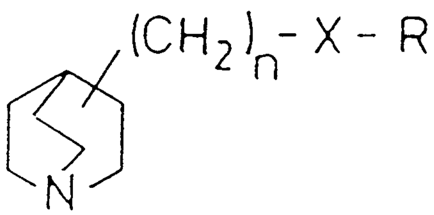

I

worin
R ein Alkylrest mit 1 bis 6 Kohlenstoffatomen, einen Alkenylrest mit 3 bis 6 Kohlenstoffatomen, einen Alkinylrest mit 3 - 6 Kohlenstoffatomen;
X Sauerstoff oder Schwefel
n = 0, 1 oder 2
gegebenenfalls in Form ihrer Racemate, ihrer Enantiomere, ihrer Diastereomere und ihrer Gemische sowie ihrer pharmakologisch unbedenklichen Säureadditionssalze wie auch ihrer Quartärsalze, - wobei 3-Methoxy-, 4-Methoxy-, 3-Ethoxy-, 3-Allyloxy-, 4-Methylthio-, 3-Methoxyethyl und 3-Methylthioethyl-1-aza-bicyclo[2,2,2]octan ausgenommen sind.

**2.** Quinuclidine der allgemeinen Formel I gemäß Anspruch I,
worin X = Sauerstoff, n = 0 oder 1 und R eine Alkylgruppe mit 1 - 3 Kohlenstoffatomen, eine Alkenylgruppe mit 3 oder 4 Kohlenstoffatomen oder eine Alkinylgruppe mit 3 oder 4 Kohlenstoffatomen, gegebenenfalls in Form ihrer Racemate, ihrer Enantiomere, ihrer Diastereomere und ihrer Gemische sowie ihrer pharmakologisch unbedenklichen Säureadditionssalze wie auch ihrer Quartärsalze.

**3.** 3-(2-Propinyloxy)-1-azabicyclo[2,2,2]octan und seine Säureadditionssalze gemäß Anspruch 1.

**4.** (-)-3-(2-Propinyloxy)-1-azabicyclo[2,2,2]octan-fumarat gemäß Anspruch 1.

5. Verwendung von Quinuclidinen der allgemeinen Formel
   I gemäß Anspruch 1 worin
   R ein Alkylrest mit 1 bis 6 Kohlenstoffatomen, einen Alkenylrest mit 3 bis 6 Kohlenstoffatomen, einen Alkinylrest mit 3 - 6 Kohlenstoffatomen;
   X Sauerstoff oder Schwefel
   n 0, 1 oder 2

   gegebenenfalls in Form ihrer Racemate, ihrer Enantiomere, ihrer Diastereomere und ihrer Gemische sowie sowie ihrer pharmakologisch unbedenklichen Säureadditionssalze wie auch ihrer Quartärsalze zur Herstellung eines Arzneimittels zur Behandlung von Krankheiten bei einer Unterfunktion des cholinergen Systems.

6. Pharmazeutische Zubereitung enthaltend eine Verbindung der allgemeinen Formel I gemäß Anspruch 1 bis 5 sowie übliche Hilfs- und/oder Trägerstoffe.

7. Verfahren zur Herstellung von pharmazeutischen Zubereitungen gemäß Anspruch 6, dadurch gekennzeichnet, daß man Verbindungen der allgemeinen Formel I mit üblichen galenischen Hilfs- und/oder Trägerstoffen mischt.

8. Verfahren zur Herstellung von Verbindung der allgemeinen Formel 1, gemäß Anspruch 1 oder 4, dadurch gekennzeichnet, daß man eine Verbindung der allgemeinen Formel

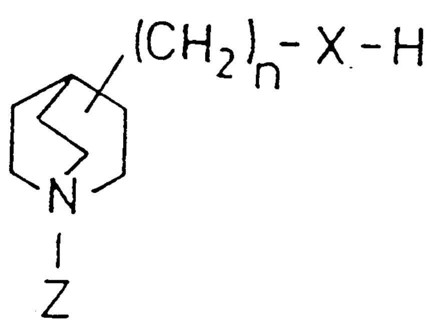

worin n und X wie zuvor definiert sind und Z eine Schutzgruppe bedeutet (im Fall von X = S, kann auf die Schutzgruppe Z verzichtet werden), deproniert und mit einem Alkylierungsreagenz der Formel

Y - R

worin R wie zuvor definiert ist und Y eine leicht abspaltbare Gruppe bedeutet umsetzt, anschließend die Schutzgruppe abspaltet und gegebenenfalls in ihrer Säureadditionssalze oder Quartärsalze überführt und/oder gegebenenfalls in ihre optisch aktiven Verbindungen trennt.

**Claims**

1. Quinuclidines of general formula I

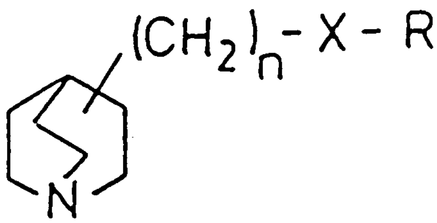

I

wherein

R denotes a $C_{1-6}$-alkyl group, a $C_{3-6}$-alkenyl group or a $C_{3-6}$-alkynyl group;
X denotes oxygen or sulphur
n = 0, 1 or 2,
optionally in the form of their racemates, enantiomers, diastereomers and the mixtures thereof, as well as the pharmacologically acceptable acid addition salts thereof and also the quaternary salts thereof, with the exception of 3-methoxy-, 4-methoxy-, 3-ethoxy-, 3-allyloxy-, 4-methylthio-, 3-methoxyethyl and 3-methylthioethyl-1-azabicyclo[2,2,2]octane.

**2.** Quinuclidines of general formula I according to claim 1, wherein X = oxygen, n = 0 or 1 and R denotes a $C_{1-3}$-alkyl group, a $C_{3-4}$-alkenyl group or a $C_{3-4}$-alkynyl group, optionally in the form of their racemates, enantiomers, diastereomers and the mixtures thereof, as well as the pharmacologically acceptable acid addition salts thereof and also the quaternary salts thereof.

**3.** 3-(2-Propynyloxy)-1-azabicyclo[2,2,2]octane and the acid addition salts thereof according to claim 1.

**4.** (-)-3-(2-Propynyloxy)-1-azabicyclo[2,2,2]octane fumarate according to claim 1.

**5.** Use of quinuclidines of general formula I according to claim 1 wherein
R denotes a $C_{1-6}$-alkyl group,
a $C_{3-6}$-alkenyl group,
a $C_{3-6}$-alkynyl group;
X denotes oxygen or sulphur
n denotes 0, 1 or 2,
optionally in the form of their racemates, enantiomers, diastereomers and the mixtures thereof, as well as the pharmacologically acceptable acid addition salts thereof and also the quaternary salts thereof, for preparing a pharmaceutical composition for treating diseases involving hypofunction of the cholinergic system.

**6.** Pharmaceutical preparation containing a compound of general formula I according to claims 1 to 5 as well as conventional excipients and/or carriers.

**7.** Process for producing pharmaceutical preparations according to claim 6, characterised in that compounds of general formula I are mixed with conventional galenic excipients and/or carriers.

**8.** Process for preparing compounds of general formula I, according to claim 1 or 4, characterised in that a compound of general formula

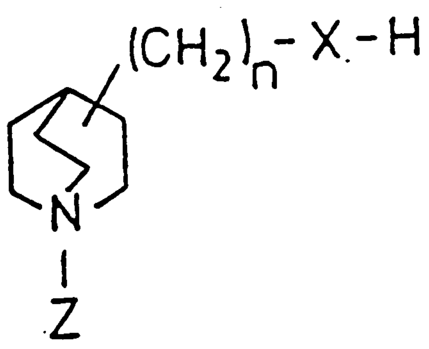

wherein n and X are as hereinbefore defined and Z denotes a protecting group (if X = S the protecting group Z can be dispensed with) is deprotonated and reacted with an alkylating reagent of formula

Y - R

wherein R is as hereinbefore defined and Y denotes a group which is easily cleaved, then the protecting group is cleaved and optionally the resulting compound is converted into an acid addition salt or quaternary salt thereof and/or optionally resolved into the optically active compounds thereof.

## Revendications

1. Quinuclidines de formule générale I

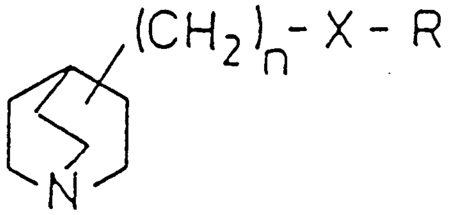

I

dans laquelle
R représente un reste alkyle de 1 à 6 atomes de carbone, un reste alcényle de 3 à 6 atomes de carbone, un reste alcynyle de 3 à 6 atomes de carbone,
X représente l'oxygène ou le soufre,
n = 0, 1 ou 2,
éventuellement sous forme de leurs racémates, de leurs énantiomères, de leurs diastéréoisomères et de leurs mélanges ainsi que de leurs sels d'addition d'acide irréprochables du point de vue pharmacologique et également de leurs sels quaternaires, à l'exception du 3-méthoxy-, 4-méthoxy-, 3-éthoxy-, 3-allyloxy-, 4-méthylthio-, 3-méthoxyéthyl- et 3-méthylthioéthyl-1-aza-bicyclo[2,2,2]-octane.

2. Quinuclidines de formule générale I selon la revendication 1, dans laquelle X = oxygène, n = 0 ou 1 et R représente un groupe alkyle de 1 à 3 atomes de carbone, un groupe alcényle de 3 ou 4 atomes de carbone ou un groupe alcynyle de 3 ou 4 atomes de carbone, éventuellement sous forme de leurs racémates, de leurs énantiomères, de leurs diastéréoisomères et de leurs mélanges ainsi que de leurs sels d'addition d'acide irréprochables du point de vue pharmacologique et de leurs sels quaternaires.

3. 3-(2-propynyloxy)-1-azabicyclo[2,2,2]octane et ses sels d'addition d'acide selon la revendication 1.

4. Fumarate de (-)-3-(2-propynyloxy)-1-azabicyclo[2,2,2]octane selon la revendication 1.

5. Utilisation de quinuclidines de formule générale I selon la revendication 1 dans lesquelles
R représente un reste alkyle de 1 à 6 atomes de carbone, un reste alcényle de 3 à 6 atomes de carbone, un reste alcynyle de 3 à 6 atomes de carbone,
X représente l'oxygène ou le soufre,
n représente 0, 1 ou 2,
éventuellement sous forme de leurs racémates, de leurs énantiomères, de leurs diastéréoisomères et de leurs mélanges ainsi que de leurs sels d'addition d'acide irréprochables du point de vue pharmacologique et de leurs sels quaternaires, pour la préparation d'un médicament pour le traitement de maladies dans le cas d'un hypofonctionnement du système cholinergique.

6. Préparation pharmaceutique contenant un composé de formule générale I selon les revendications 1 à 5 ainsi que des adjuvants et/ou véhicules courants.

7. Procédé de préparation de préparations pharmaceutiques selon la revendication 6, caractérisé en ce que l'on mélange des composés de formule générale I avec des adjuvants et/ou véhicules galéniques courants.

8. Procédé de préparation d'un composé de formule générale I selon la revendication 1 ou 4, caractérisé en ce que l'on déprotone un composé de formule générale

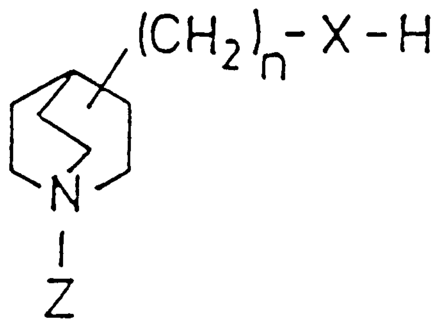

dans laquelle n et X sont définis comme précédemment et Z représente un groupe protecteur (dans le cas où X = S, il est possible de renoncer au groupe protecteur Z) et on le fait réagir avec un réactif d'alkylation de formule

Y - R

dans laquelle R est défini comme précédemment et Y représente un groupe facilement éliminable, puis on élimine le groupe protecteur et on convertit éventuellement le composé en ses sels d'addition d'acide ou en ses sels quaternaires et/ou on le résout éventuellement en ses composés optiquement actifs.